# EUROPEAN PATENT SPECIFICATION

(11) **EP 3 895 745 B1**
(45) Date of publication and mention of the grant of the patent: **25.12.2024**
(21) Application number: 19888264.9
(22) Date of filing: 11.10.2019
(51) Int. Cl.: A61M 1/00, A61B 5/03, A61B 1/00, A61B 1/015, A61B 1/07, G16H 20/40, G16H 30/40

(54) **INTELLIGENT INTRACRANIAL HEMATOMA REMOVAL AND DRAINAGE SYSTEM**
INTELLIGENTES SYSTEM ZUR ENTFERNUNG UND DRAINAGE INTRAKRANIELLER HÄMATOME
SYSTÈME INTELLIGENT D'ÉLIMINATION ET DE DRAINAGE D'HÉMATOME INTRACRÂNIEN

(30) Priority: 28.11.2018 CN 201811430652
(43) Date of publication of application: 20.10.2021
(73) Proprietor: Beijing Wantefu Medical Apparatus Co., Ltd, Beijing 102200 (CN)
(72) Inventor: LIU, Wenjie, Beijing 102200 (CN); CUI, Shaofei, Beijing 102200 (CN); TANG, Zhouping, Beijing 102200 (CN); YANG, Shirun, Beijing 102200 (CN); LI, Yuanwei, Beijing 102200 (CN); WU, Guofeng, Beijing 102200 (CN); YANG, Qingwu, Beijing 102200 (CN); ZHAO, Zhizeng, Beijing 102200 (CN); LI, Yu, Beijing 102200 (CN)
(74) Representative: Cleanthous, Marinos
(86) International application number: PCT/CN2019/110566
(87) International publication number: WO 2020/108112

(56) References cited:
- WO-A1-2018/035366
- CN-A- 1 220 136
- CN-A- 104 225 780
- CN-A- 108 853 618
- CN-A- 108 853 621
- CN-A- 109 663 153
- CN-U- 202 666 054
- CN-U- 203 620 060
- US-A1- 2013 226 066

## Description

### TECHNICAL FIELD

The present disclosure relates to the technical field of medical instruments, and in particular to an intelligent intracranial hematoma removal and drainage system, which is suitable for removing intracranial hematoma and draining in a cerebral ventricle.

### BACKGROUND

Intracerebral hemorrhage is a common and frequently-occurring disease, with acute onset, high mortality and high disability rate. It is particularly important in clinical treatment to remove intracranial hematoma in time and reduce intracranial pressure.

Main methods for treating intracerebral hemorrhage include medical conservative treatment, hematoma removal through craniotomy and hematoma removal through minimally invasive puncture, especially hematoma removal through minimally invasive puncture, which has recently been proved by RCT clinical research to be an effective method for treating intracranial hematoma. However, in clinical application, this method has problems such as the need for regular suction, douching, liquifying and draining of intracranial hematoma repeatedly done by a doctor, a long time of the process and the dependence on the doctors' personal experience.

In view of the problems in related technologies, no effective solutions have been proposed yet.

CN108853618 discloses a human body cavity drainage measuring and controlling device. The device is mainly characterized in that the drainage flow from human body cavity drainage can be monitored inreal time, data of the drainage flow can be transmitted out in a wireless transmission mode, and in addition, the human body cavity drainage flow can be controlled according to clinical needs; the device mainly comprises a sensor unit capable of collecting the drainage flow data, an integrated control circuit, a wireless data transmission module, a flow regulation control mechanism, a drainage pipeline, a power source and other structures.

### SUMMARY

In view of the aforementioned technical problems in related technologies, the present disclosure provides an intelligent intracranial hematoma removal and drainage system, which can not only replace manual long-term monitoring, but also formulate an optimal treatment operation scheme according to the sign detection results of a patient, thereby increasing the safety, normativity and effectiveness of treatment.

In order to achieve the aforementioned technical objective, the technical solution of the present disclosure is realized as follows.

Disclosed is an intelligent intracranial hematoma removal and drainage system, including: an information input and record module, an information detection module, an intelligent module and a hematoma removal and drainage module, wherein
the information input module is used for collecting preoperative data and transmitting the collected preoperative data to the intelligent module;
the information detection module is used for acquiring intracranial data monitored in real time and transmitting the monitored intracranial data to the intelligent module;
the intelligent module is used for receiving the data from the information input module and the information detection module, analyzing the received data to make a determination, and converting the determination into an instruction signal and transmitting the same to the hematoma removal and drainage module; and
the hematoma removal and drainage module is used for receiving an instruction from the intelligent module to remove and drain intracranial hematomas, and feeding data after removal and drainage back to the intelligent module.

Further, the information detection module includes an image detection module, a signal acquisition module and a drainage detection module, wherein
the image detection module is used for detecting the condition of the intracranial hematoma and determine the shape of the intracranial hematoma;
the signal acquisition module is used for acquiring intracranial pressure information and monitoring and transmitting the pressure information in real time; and
the drainage detection module is used for detecting drainage components through real-time analysis, detecting the contents of blood components and monitoring rebleeding in real time.

Further, the signal acquisition module includes an intracranial pressure sensor and a signal conditioning module, the intracranial pressure sensor is in communication connection with the signal conditioning module, and the signal conditioning module is used for performing nonlinear amplification processing on the intracranial pressure sensor and transmitting the conditioned signal to the hematoma removal and drainage module for operation.

Further, the drainage detection module includes a spectral detection module and a color detection module, which are respectively transmitted to a main control system, wherein the spectral detection module is used for detecting spectral information; and the color detection module is used for detecting color information.

Further, the intelligent module includes a cloud database, an intelligent analysis module, a data processing module and an instruction signal transmission module, wherein
the cloud database is used for classifying and storing data and updating the stored data in real time;
the intelligent analysis module is used for comparing and analyzing various collected data with the original data in the cloud database;
the data processing module is used for receiving intracranial data and transmitting the intracranial data in real time; and
the instruction signal transmission module is used for transmitting the instruction signal.

Further, the information input module includes a basic information module and a pathogenetic condition description module, wherein the basic information module is used for inputting the basic information of the patient; and the pathogenetic condition description module is used for inputting the basic description of the pathogenetic condition of the patient.

Further, the data of the basic information module includes but is not limited to the patient's name, gender, age, native place and medical record number.

Further, the treatment modes of the hematoma removal and drainage module include, but are not limited to, a simple negative-pressure suction mode, a simple pressureless drainage mode, an isobaric replacement and drainage mode, a drip-irrigation replacement and drainage mode, and a mode of injecting a medical solution through a drainage clamping channel.

Further, the system further includes an alarm module and a power supply module, wherein
the alarm module is used for providing an alarm when manual intervention is needed or the information input and record module, the information detection module, the intelligent module and the hematoma removal and drainage module fail; and
the power supply module is used for supplying direct-current voltages to the information input and record module, the information detection module, the intelligent module and the hematoma removal and drainage module, respectively.

Beneficial effects of the present disclosure:
1. not only corresponding bases are provided for all treatment operations, but also all signals are detected in real time, thereby ensuring the real-time monitoring of key information of a patient during an intracranial hematoma treatment process, such that all the treatment operations on the patient are controllable and precise; and
2. It can not only replace manual long-term monitoring, but also formulate an optimal treatment operation scheme according to the sign detection results of a patient, thereby increasing the safety, normativity and effectiveness of treatment.

### BRIEF DESCRIPTION OF THE DRAWINGS

To describe the technical solutions in the embodiments of the present disclosure or in the prior art more clearly, the following briefly introduces the accompanying drawings required for describing the embodiments. Apparently, the accompanying drawings in the following description show merely some embodiments of the present disclosure, and those of ordinary skills in the art may still derive other drawings from these accompanying drawings without creative efforts.
FIG. 1 is a schematic diagram of an intelligent intracranial hematoma removal and drainage system according to an embodiment of the present disclosure;
FIG. 2 is one of the schematic diagrams of a hematoma removal and drainage module according to an embodiment of the present disclosure;
FIG. 3 is a schematic diagram of a signal acquisition module according to an embodiment of the present disclosure;
FIG. 4 is a schematic diagram of a drainage detection module according to an embodiment of the present disclosure;
FIG. 5 is a second one of schematic diagrams of a hematoma removal and drainage module according to an embodiment of the present disclosure; and
FIG. 6 is a schematic diagram of a panel structure according to an embodiment of the present disclosure.

### DESCRIPTION OF THE EMBODIMENTS

The following clearly and completely describes the technical solutions in the embodiments of the present disclosure with reference to the accompanying drawings in the examples of the present disclosure. Apparently, the described examples are merely a part rather than all of the embodiments of the present disclosure. All other embodiments obtained by those of ordinary skills in the art based on the embodiments of the present disclosure are within the claimed scope of the present disclosure. The invention is defined by the appended claims.

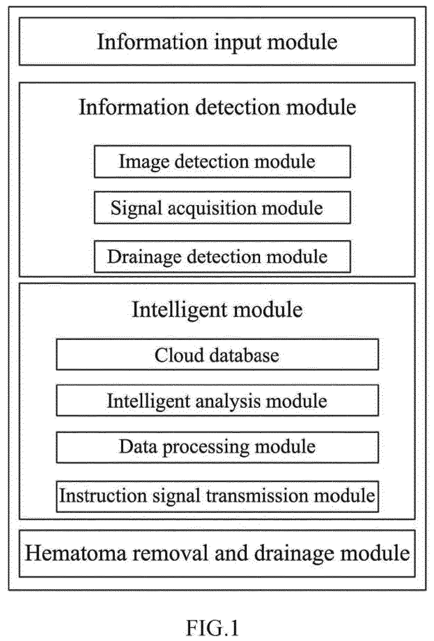

As shown in FIG. 1, an intelligent intracranial hematoma removal and drainage system according to an embodiment of the present disclosure includes an information input and record module, an information detection module, an intelligent module and a hematoma removal and drainage module, wherein
the information input module is used for collecting preoperative data and transmitting the collected preoperative data to the intelligent module;
Specifically, the information input module has the functions of inputting and uploading the preoperative information of the patient (e.g., the pathogenetic condition, CT photographs, detection results, etc.) to a cloud database, searching and comparing for the intelligent module to call.
the information detection module is used for acquiring intracranial data monitored in real time and transmitting the monitored intracranial data to the intelligent module;
the intelligent module is used for receiving the data from the information input module and the information detection module, analyzing the received data to make determination, and converting the determination into an instruction signal and transmitting the same to the hematoma removal and drainage module; and
the hematoma removal and drainage module is used for receiving an instruction from the intelligent module to remove and drain intracranial hematomas, and feeding data after removal and drainage back to the intelligent module.

In a specific embodiment of the present disclosure, the information detection module includes an image detection module, a signal acquisition module and a drainage detection module, wherein
the image detection module is used for detecting the condition of the intracranial hematoma and determine the shape of the intracranial hematoma,
specifically, the image detection module can choose an endoscope system, which is composed of an endoscope, a camera, an image storage, an illumination source and an image data transmission device, the enclosure package of the endoscope adopts stainless-steel hard straight tubes, and a superfine cerebral ventricle endoscope is selected as the endoscope, wherein an internal light transmission structure is internal and external layers of light transmission media, and the internal light transmission medium adopts an image transmission fiber with more than 30,000 pixels and is mainly used for the transmission of image pixels; the external light transmission medium is a flexible single-strand glass fiber, which is wrapped on the internal image transmission fiber and used for transmitting to the illumination source, the endoscope is connected to the intelligent intracranial hematoma removal and drainage system through a connecting cable to display images, the endoscope system has the functions of illumination, and real-time observation, storage and transmission of images, and is used for observing intracranial hematoma, the endoscope system mainly realizes the observation of hematoma state, so as to provide a basis for the intelligent system to intelligently calculate a corresponding treatment scheme;
the signal acquisition module is used for acquiring intracranial pressure information and monitoring and transmitting the pressure information in real time; and
the drainage detection module is used for detecting drainage components through real-time analysis, detecting the contents of blood components and monitoring rebleeding in real time.

Specifically, the aforementioned rebleeding includes micro-bleeding, and the drainage detection module has the functions of monitoring the content of the drainage in blood in real time, finding intracranial fresh micro-bleeding or rebleeding in real time, and online detecting markers the intracranial drainage which have guiding significance for treatment in.

In a specific embodiment of the present disclosure, the signal acquisition module includes an intracranial pressure sensor and a signal conditioning module, the intracranial pressure sensor is in communication connection with the signal conditioning module, and the signal conditioning module is used for performing nonlinear amplification processing on the intracranial pressure sensor and transmitting the conditioned signal to the hematoma removal and drainage module for operation.

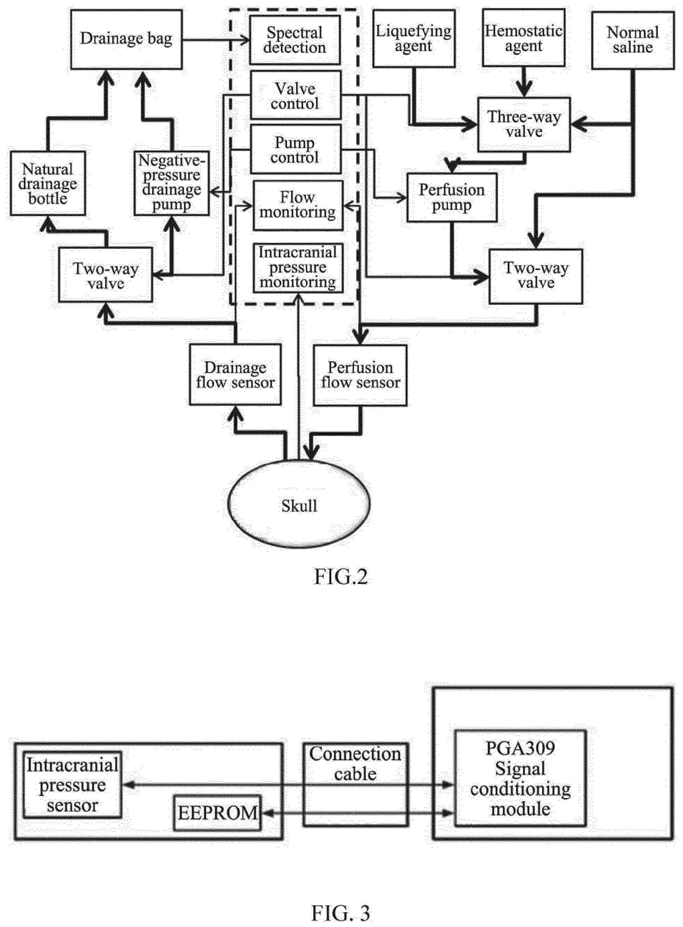

Specifically, as shown in FIG. 3, the signal acquisition module is an intracranial pressure signal acquisition device, which includes an intracranial pressure sensor, a connecting cable and a PGA signal conditioning module. The intracranial pressure sensor is a miniature implantable intracranial pressure sensor, the intracranial pressure signal acquisition device is composed of a miniature piezoresistive induction wafer (e.g., P162) and a memory chip EEPROM. The miniature piezoresistive induction wafer adopts a half-bridge circuit for output, and the outer diameter of the package shell is no larger than 1.8 mm through a packaging process; then the intracranial pressure sensor is connected to the PGA signal conditioning module through the connecting cable, the PGA signal conditioning module adopts a programmable logic amplifier (e.g., PGA309) to perform nonlinear amplification treatment on a differential pressure signal output by the intracranial pressure sensor after a full-bridge circuit is complemented, and is connected to the EEPROM memory chip of the sensor module, so as to write calibrated sensor parameter information into the chip. Finally, the conditioned signal is accessed into the intelligent cerebral ventricle hematoma removal and drainage system for A/D conversion, wave filtering, displaying and the like operations. The intracranial pressure signal acquisition device mainly achieves monitoring of the intracranial pressure signal, and provides the basis for the change of the subsequent hematoma removal and drainage speed.

In a specific embodiment of the present disclosure, the drainage detection module includes a spectral detection module and a color detection module, which are respectively transmitted to a main control system, wherein the spectral detection module is used for detecting spectral information; and the color detection module is used for detecting color information.

Specifically, the spectral detection module adopts a spectrometer for detection; wherein, the spectral range is 350 nm-1,000 nm, the resolution is about 15 nm at 25 µm, the spectral sampling interval is about 0.35 nm, and the light source adopts a halogen tungsten lamp with the wavelength of 360-2,400 nm; the color detection module adopts a TCS34725 module, of which the working principle is: an illumination LED emits light which irradiates an object to be detected, the returned light is detected by a filter for an RGB proportional value, and the color is identified according to the RGB proportional value.

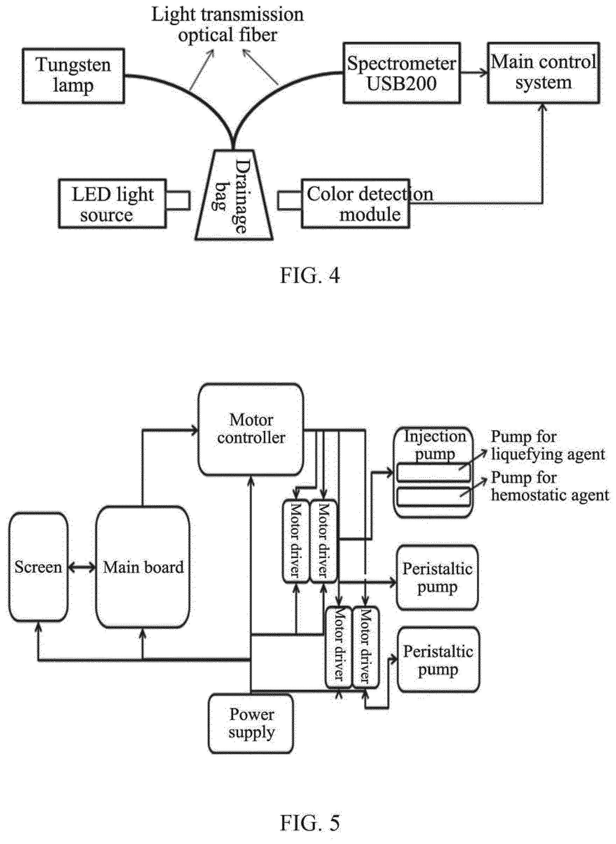

As shown in FIG. 4, the drainage detection module specifically includes a spectrometer USB2000, a tungsten lamp, a light transmission optical fiber, an LED light source, a color detection module and a data connection line, etc. The tungsten lamp emits light with the wavelength of 360-2,400 nm, which irradiates the drainage precipitate in a drainage bag through the optical fiber, and then the light is transmitted to the spectrometer USB2000 through a reflective optical fiber, and finally the spectral information is transmitted to the main control system through the data connection line for analysis. An LED light source at the other end irradiates the drainage in the drainage bag, and then the light is transmitted to the color detection module, and the color detection module transmits the detected color information to the main control system through the data connection line.

In a specific embodiment of the present disclosure, the intelligent module includes a cloud database, an intelligent analysis module, a data processing module and an instruction signal transmission module, wherein
the cloud database is used for classifying and storing data and updating the stored data in real time;
the intelligent analysis module is used for comparing and analyzing various collected data with the original data in the cloud database;
the data processing module is used for receiving intracranial data and transmitting the intracranial data in real time; and
the instruction signal transmission module is used for transmitting the instruction signal.

Specifically, the intelligent module includes an intelligent analysis module, a cloud database, a data processing module and an instruction signal sending device. The data processing module is a data receiving and transmitting device, and the main working mode of it is: the received data is transmitted to the cloud database in real time and then classified and stored. The intelligent analysis module compares and analyzes the uploaded data with the original data of the cloud database to make determination, convert the determination into an instruction signal, and sends the instruction signal to a corresponding system, so as to control the corresponding system to work according to the instruction.

The intelligent module has the function of giving the treatment mode by comparing and analyzing the data of the image detection module, the signal acquisition module and the information input module respectively with that of the cloud database; the function of giving corresponding subsequent treatment operations by determining the intracranial change of a patient (such as whether the hematoma becomes smaller, whether there is fresh micro-bleeding or rebleeding, changes of intracranial pressure, and the like conditions) by the intelligent module according to the information given by the information detection module during the treatment process of hematoma removal, until the expected hematoma removal effect is achieved; and the function of giving an alarm when it is determined that manual intervention is needed or system failure occurs.

The intelligent module gives a treatment mode for a doctor to choose by comparing and analyzing the aforementioned data with the data set in the cloud database through the intelligent analysis module. After the treatment mode is selected, the system starts the first intelligent intracranial hematoma removal according to the intracranial pressure condition.

In a specific embodiment of the present disclosure, the information input module includes a basic information module and a pathogenetic condition description module, wherein the basic information module is used for inputting the basic information of the patient; and the pathogenetic condition description module is used for inputting the basic description of the pathogenetic condition of the patient.

Specifically, it includes a basic information module and a pathogenetic condition description module, wherein the information of the basic information module adopts a standard input mode, which is convenient for the doctor to input the basic information of the patient such as the name, gender, age, native place, medical record number and the like before a hematoma removal surgery. The pathogenetic condition description module is used for filling in the basic description of the pathogenetic condition after the doctor enters the basic information of the patient, which provides more basis for the intelligent module to give the treatment mode subsequently after deep learning and comparing in the cloud database.

In a specific embodiment of the present disclosure, the treatment modes of the hematoma removal and drainage module include, but are not limited to, a simple negative-pressure suction mode, a simple pressureless drainage mode, an isobaric replacement and drainage mode, a drip-irrigation replacement and drainage mode, and a mode of injecting a medical solution by clamping the drainage channel.

Specifically, the hematoma removal and drainage module is provided with an independent liquid inlet channel, a drainage channel and an administration channel; has simple negative-pressure suction mode, a simple pressureless drainage mode, an isobaric replacement and drainage mode in which the drainage is inlet quantitatively in the liquid inlet channel while the drainage channel is subjected to equivalent suction, a drip-irrigation replacement and drainage mode in which the drainage is inlet through drip-irrigation in the liquid inlet channel while the drainage channel is subjected to equivalent drainage, and a mode of injecting a medical solution by clamping the drainage channel, and selects the aforementioned treatment mode to remove the intracranial hematoma through the signal given by the intelligent module; and has the functions of uploading working parameters to the intelligent module in real time and receiving a signal from the intelligent module in real time.

More specifically, it includes an independent liquid inlet channel, a drainage channel, an administration channel, an administration device, a data collection and transmission system, a motor and a motor controller, a peristaltic pump, a pinch valve and a main control system. The main application mode is that: the main control system (e.g., Smart210) sends a control instruction to the motor controller according to the signal given by the intelligent module, and the motor controller controls the running of the peristaltic pump or the administration device and the on-off of the pinch valve, wherein it is required that the minimum volume accuracy of drainage, liquid inlet and administration is no more than 0.1 mL, the minimum drainage speed is no more than 1 mL/min, the maximum drainage speed is no less than 5 mL/min, and the liquid volume error of each drainage and liquid inlet is no more than 2%.

The aforementioned administration device is realized by a high-precision injection pump. The high-precision injection pump has a minimum administration amount of no more than 0.5 mL and a maximum administration amount of no less than 10 mL under the administration mode. For the injection, the minimum liquid inlet speed is no more than 0.1 mL/min, the maximum liquid inlet speed is no less than 2 mL/min, and the volume error of each injection is no more than 2%.

As shown in FIG. 2, the aforementioned administration device is mainly used for administrating two agents of a liquefying agent and a hemostatic agent. The liquefying agent, the hemostatic agent and normal saline are three kinds of inletting liquid. The three liquid enter a perfusion pump through a three-way valve, and meanwhile normal saline enters a perfusion flow sensor through a two-way valve between the perfusion pump, and then enter the skull of the patient. The drainage liquid first passes through a drainage flow sensor, then enters the two-way valve to enter a negative-pressure drainage pump and a natural drainage bottle, and finally all of them are collected into a drainage bag; wherein, the intracranial pressure sensor (for monitoring the intracranial pressure) is implanted into the skull of the patient to monitor the intracranial physiological parameters in real time, and the flow sensor (for monitoring the flow) monitors the inflow and outflow of the drainage in real time. Finally, the drainage bag also determines the current hematoma state through spectral detection (spectral analysis and color detection), and controls the pump and the pinch valve (pump control and valve control) in real time to adjust the treatment operation.

As shown in FIG. 5, the hematoma removal and drainage module includes a removal and drainage part and a two-way administration module. Firstly, the main control system uses Smart210 based on an embedded linux system to conduct data analysis of the whole system, and connected with a screen through a HDMI data line for displaying and corresponding controlling. The main control system is connected with the motor controller through RS232, and transmits the control instruction for the motor and the control instruction for the pinch valve to 4 stepping motor drivers, wherein two stepping motor drivers are connected with the drainage peristaltic pump and the liquid inlet pump, and the other two stepping motor drivers are connected with the injection pump of the two-way administration device.

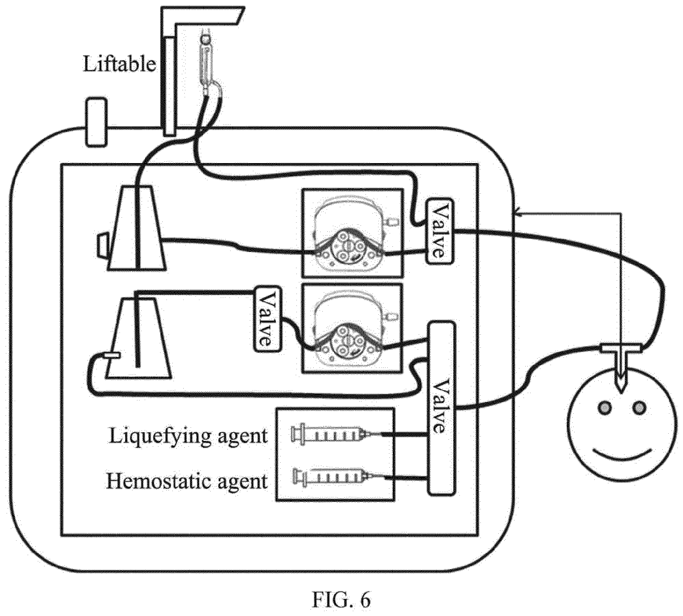

As shown in FIG. 6, the upper part is a drainage path, and the lower part is a liquid inlet pipe. Normal saline is connected to the skull through a liquid inlet pump, which can realize drip irrigation/replacement and the like operations, and then a liquid outlet pipe is connected with a liquid outlet pump and a natural drainage bottle to realize negative-pressure drainage, natural drainage, and the like operations. At the bottom is the schematic diagram of the injection pump of the two-way administration device, which adopts a standard syringe. The doctor fills the syringe with a medical solution and then installs it at the corresponding injection pump position to conduct preparation operations for administration.

In a specific embodiment of the present disclosure, the system further includes an alarm module and a power supply module, wherein
the alarm module is used for providing an alarm when manual intervention is needed or the information input and record module, the information detection module, the intelligent module and the hematoma removal and drainage module fail; and
the power supply module is used for supplying direct-current voltages to the information input and record module, the information detection module, the intelligent module and the hematoma removal and drainage module, respectively.

Specifically, the alarm module has the function of prompting alarm when manual intervention is needed or system failure occurs during the treatment process. The alarm module includes an audible and visual alarm and an alarm turning-off device, and is mainly used for alarming when the physiological parameters of the patient have uncontrollable changes during the treatment process, so as to remind the doctor to carry out manual intervention.

The power supply module has the function of providing direct current for the information input and record module, the information detection module, the intelligent module and the hematoma removal and drainage module, and has the function of independently providing direct-current power supply for the information input and record module, the information detection module, the intelligent module and the hematoma removal and drainage module for no less than 6 hours in case of unexpected power down of the external alternating current, so as to ensure that the information input and record module, the information detection module, the intelligent module and the hematoma removal and drainage module continue to fail normally without external power. The power supply circuit in the power supply module includes a transformer and a linear voltage-regulator tube; The alternating current is converted into direct current through the transformer and a rectifier filter, and then provides the required direct current voltage respectively for the information input and record module, the information detection module, the intelligent module and the hematoma removal and drainage module through the linear voltage-regulator tube.

In order to facilitate the understanding of the aforementioned technical solution of the present disclosure, the aforementioned technical solution of the present disclosure will be described in detail in terms of specific usage mode below.

In the specific use, according to the intelligent intracranial hematoma removal and drainage system of the present disclosure, after puncture is completed, the condition of intracranial hematoma is observed and the hematoma shape is determined through the image detection module, the intracranial pressure information is obtained by the signal acquisition module, and the treatment mode is given by the intelligent module for the doctor to choose according to the hematoma shape, the intracranial pressure and the pathogenetic condition information of the patient. After the working mode is selected, the system starts the first intelligent intracranial hematoma removal according to the intracranial pressure condition. The first removal is completed after the intracranial pressure drops to the set value, and then the operations such as replacement drainage/or drip irrigation drainage/or clamping and injection of hematoma liquefying agent are carried out according to the treatment mode. In this process, in the system, the intelligent module determines the intracranial changes of the patient (such as whether the hematoma becomes smaller, whether there is fresh micro-bleeding or rebleeding, changes of the intracranial pressure, and the like conditions) according to the information given by the image detection module, signal acquisition module and drainage detection module, and thus gives corresponding subsequent treatment operation steps until the preset hematoma removal effect is achieved, wherein the intracranial changes of the patient specifically include, for example, whether the hematoma becomes smaller, whether there is fresh micro-bleeding or rebleeding, and changes of the intracranial pressure, etc.

### Specific working manner:

1. the condition of intracranial hematoma after the puncture is observed through the endoscope system so as to determine the hematoma shape and the like information. There are two working modes: an automatic mode, in which the endoscope camera system automatically transmits the image to the intelligent module, which determines the hematoma shape and the like information; and a manual mode, in which the doctor observes the image and makes determination, and then the image, the determination conclusion and the like information are transmitted to the intelligent module. The intracranial pressure information is acquired through the intracranial pressure signal acquisition device, the pressure signal is processed to display the waveform, and the intracranial pressure information is transmitted to the intelligent system in real time; and the pathogenetic condition information of the patient is transmitted to the intelligent module through the information input module;
2. the intelligent module gives a treatment mode for a doctor to choose by comparing and analyzing the aforementioned data with the data set in the cloud database through the intelligent analysis module;
3. after the treatment mode is selected, the hematoma removal and drainage module start the first intelligent intracranial hematoma removal according to the intracranial pressure condition, the first removal is completed after the intracranial pressure drops to the set value, and then the operations such as replacement drainage/or drip irrigation drainage/or clamping and injection of hematoma liquefying agent are carried out according to the treatment mode;
4. during this process, in the system, the intelligent module determines the intracranial changes of the patient (such as whether the hematoma becomes smaller, whether there is fresh micro-bleeding or rebleeding, changes of the intracranial pressure, and the like conditions) according to the information given by the drainage detection module, the intracranial pressure signal acquisition device and the endoscope system, and thus gives corresponding subsequent treatment operation steps until the preset hematoma removal effect is achieved;
5. when the intelligent module judges that the treatment needs manual intervention or system failure occurs, the alarm module gives an alarm; and
6. the power supply module provides the required direct-current voltages for the intracranial pressure signal acquisition device, the endoscope system, the information input module, the drainage detection module, the hematoma removal and drainage module, the intelligent module and the alarm module.

In view of the above, by means of the aforementioned technical solution of the present disclosure, the system is safe and effective, makes the hematoma removal treatment process be precise and intelligent, and reduces medical accidents caused by human factors such as improper operation of the doctor. Only by connecting individual signal detection modules to the patient, the system can accurately give the follow-up treatment according to the current state signal of the patient, and monitor the patient in real time and adjust the treatment mode in time during the treatment process, thereby forming a closed-loop treatment system and realizing the functions of long-term monitoring and automatic treatment of the intraventricular hematoma.

## Claims

1. An intelligent intracranial hematoma removal and drainage system, comprising an information input and record module, an information detection module, an intelligent module and a hematoma removal and drainage module, wherein
the information input module is used for collecting preoperative data and transmitting the collected preoperative data to the intelligent module;
the information detection module is used for acquiring intracranial data monitored in real time and transmitting the monitored intracranial data to the intelligent module;
the intelligent module is used for receiving the data from the information input module and the information detection module, analyzing the received data to make a determination, and converting the determination into an instruction signal and transmitting the same to the hematoma removal and drainage module; and
the hematoma removal and drainage module is used for receiving an instruction from the intelligent module to remove and drain intracranial hematomas, and feeding data after removal and drainage back to the intelligent module, the intelligent intracranial hematoma removal and drainage system is characterized that,
the information detection module comprises an image detection module, a signal acquisition module and a drainage detection module, wherein
the image detection module is used for detecting the condition of the intracranial hematoma and determine the shape of the intracranial hematoma;
the signal acquisition module is used for acquiring intracranial pressure information and monitoring and transmitting the pressure information in real time; and
the drainage detection module is used for detecting drainage components through real-time analysis, detecting the contents of blood components and monitoring rebleeding in real time.

2. The intelligent intracranial hematoma removal and drainage system according to claim 1, wherein the signal acquisition module comprises an intracranial pressure sensor and a signal conditioning module, the intracranial pressure sensor is in communication connection with the signal conditioning module, and the signal conditioning module is used for performing nonlinear amplification processing on the intracranial pressure sensor and transmitting the conditioned signal to the hematoma removal and drainage module for operation.

3. The intelligent intracranial hematoma removal and drainage system according to claim 1, wherein the drainage detection module comprises a spectral detection module and a color detection module, which respectively transmit data to a main control system, wherein the spectral detection module is used for detecting spectral information; and the color detection module is used for detecting color information.

4. The intelligent intracranial hematoma removal and drainage system according to claim 1, wherein the intelligent module comprises a cloud database, an intelligent analysis module, a data processing module and an instruction signal transmission module, wherein
the cloud database is used for classifying and storing data and updating the stored data in real time;
the intelligent analysis module is used for comparing and analyzing various collected data with the original data in the cloud database;
the data processing module is used for receiving intracranial data and transmitting the intracranial data in real time; and
the instruction signal transmission module is used for transmitting the instruction signal.

5. The intelligent intracranial hematoma removal and drainage system according to claim 1, wherein the information input module comprises a basic information module and a pathogenetic condition description module, wherein the basic information module is used for inputting the basic information of the patient; and the pathogenetic condition description module is used for inputting the basic description of the pathogenetic condition of the patient.

6. The intelligent intracranial hematoma removal and drainage system according to claim 5, wherein the data of the basic information module comprises but is not limited to the patient's name, gender, age, native place and medical record number.

7. The intelligent intracranial hematoma removal and drainage system according to claim 1, wherein the treatment modes of the hematoma removal and drainage module comprise, but are not limited to, a simple negative-pressure suction mode, a simple pressureless drainage mode, an isobaric replacement and drainage mode, a drip-irrigation replacement and drainage mode, and a mode of injecting a medical solution through a drainage clamping channel.

8. The intelligent intracranial hematoma removal and drainage system according to any one of claims 1-7, further comprising an alarm module and a power supply module, wherein
the alarm module is used for providing an alarm when manual intervention is needed or the information input and record module, the information detection module, the intelligent module and the hematoma removal and drainage module fail; and
the power supply module is used for supplying direct-current voltages to the information input and record module, the information detection module, the intelligent module and the hematoma removal and drainage module, respectively.

## Patentansprüche

1. Intelligente System zur Entfernung und Drainage intrakranieller Hämatome, umfassend ein Informationseingabe- und -aufzeichnungsmodul, ein Informationserkennungsmodul, ein intelligentes Modul und ein Hämatomentfernungs- und -drainagemodul, wobei das Informationseingabemodul zum Sammeln von präoperativen Daten und zum Übertragen der gesammelten präoperativen Daten an das intelligente Modul dient;
das Informationserkennungsmodul zum Erfassen von in Echtzeit überwachten intrakraniellen Daten und zum Übertragen der überwachten intrakraniellen Daten an das intelligente Modul dient;
das intelligente Modul zum Empfangen der Daten vom Informationseingabemodul und dem Informationserkennungsmodul, zum Analysieren der empfangenen Daten, um eine Bestimmung vorzunehmen, zum Umwandeln der Bestimmung in ein Anweisungssignal und zum Übertragen desselben an das Hämatomentfernungs- und -drainagemodul dient; und
das Hämatomentfernungs- und -drainagemodul zum Empfangen einer Anweisung vom intelligenten Modul, um intrakranielle Hämatome zu entfernen und zu drainieren, und zum Einspeisen von Daten nach der Entfernung und Drainage zurück in das intelligente Modul dient, **dadurch gekennzeichnet, dass**
das Informationserkennungsmodul ein Bilderkennungsmodul, ein Signalerfassungsmodul und ein Drainageerkennungsmodul umfasst, wobei
das Bilderkennungsmodul zur Erkennung des Zustands des intrakraniellen Hämatoms und zur Bestimmung der Form des intrakraniellen Hämatoms dient;
das Signalerfassungsmodul zum Erfassen von intrakraniellen Druckinformationen und zum Überwachen und Übertragen der Druckinformationen in Echtzeit dient; und
das Drainageerkennungsmodul zur Erkennung von Drainagekomponenten durch Echtzeitanalyse, zur Erkennung des Inhalts von Blutkomponenten und zur Überwachung von Nachblutungen in Echtzeit dient.

2. Intelligente System zur Entfernung und Drainage intrakranieller Hämatome nach Anspruch 1, wobei das Signalerfassungsmodul einen intrakraniellen Drucksensor und ein Signalaufbereitungsmodul umfasst, wobei der intrakranielle Drucksensor in Kommunikationsverbindung mit dem Signalaufbereitungsmodul steht, und wobei das Signalaufbereitungsmodul zur Durchführung einer nichtlinearen Verstärkungsverarbeitung des intrakraniellen Drucksensors und zur Übertragung des aufbereiteten Signals an das Hämatomentfernungs- und -drainagemodul für den Betrieb verwendet wird.

3. Intelligente System zur Entfernung und Drainage intrakranieller Hämatome nach Anspruch 1, wobei das Drainageerkennungsmodul ein Spektralerkennungsmodul und ein Farberkennungsmodul umfasst, die jeweils Daten an ein Hauptsteuersystem übertragen, wobei das Spektralerkennungsmodul zum Erkennen von Spektralinformationen verwendet wird und das Farberkennungsmodul zum Erkennen von Farbinformationen verwendet wird.

4. Intelligente System zur Entfernung und Drainage intrakranieller Hämatome nach Anspruch 1, wobei das intelligente Modul eine Cloud-Datenbank, ein intelligentes Analysemodul, ein Datenverarbeitungsmodul und ein Anweisungssignalübertragungsmodul umfasst, wobei die Cloud-Datenbank zum Klassifizieren und Speichern von Daten und zum Aktualisieren der gespeicherten Daten in Echtzeit verwendet wird;
das intelligente Analysemodul zum Vergleichen und zur Analyse verschiedener gesammelter Daten mit den Originaldaten in der Cloud-Datenbank verwendet wird;
das Datenverarbeitungsmodul zum Empfangen intrakranieller Daten und zum Übertragen der intrakraniellen Daten in Echtzeit verwendet wird; und
das Anweisungssignalübertragungsmodul zur Übertragung des Anweisungssignals verwendet wird.

5. Intelligente System zur Entfernung und Drainage intrakranieller Hämatome nach Anspruch 1, wobei das Informationseingabemodul ein Basisinformationsmodul und ein Modul zur Beschreibung des pathogenetischen Zustands umfasst, wobei das Basisinformationsmodul zur Eingabe der Basisinformationen des Patienten verwendet wird; und das Modul zur Beschreibung des pathogenetischen Zustands zur Eingabe der Basisbeschreibung des pathogenetischen Zustands des Patienten verwendet wird.

6. Intelligente System zur Entfernung und Drainage intrakranieller Hämatome nach Anspruch 5, wobei die Daten des Basisinformationsmoduls den Namen, das Geschlecht, das Alter, den Geburtsort und die Krankenaktennummer des Patienten umfassen, aber nicht darauf beschränkt sind.

7. Intelligente System zur Entfernung und Drainage intrakranieller Hämatome nach Anspruch 1, wobei die Behandlungsmodi des Hämatom-Entfernungs- und Drainagemoduls unter anderem einen einfachen Unterdruck-Saugmodus, einen einfachen drucklosen Drainagemodus, einen isobaren Austausch- und Drainagemodus, einen Tropfspülungsaustausch- und Drainagemodus und einen Modus zur Injektion einer medizinischen Lösung durch einen Drainageklemmkanal umfassen.

8. Intelligente System zur Entfernung und Drainage intrakranieller Hämatome nach einem der Ansprüche 1 bis 7, umfassend außerdem ein Alarmmodul und ein Stromversorgungsmodul, wobei
das Alarmmodul verwendet wird , um einen Alarm bereitzustellen, wenn ein manueller Eingriff erforderlich ist oder das Informationseingabe- und -aufzeichnungsmodul, das Informationserkennungsmodul, das intelligente Modul und das Hämatomentfernungs- und - drainagemodul ausfallen; und
das Stromversorgungsmodul zur Versorgung des Informationseingabe- und - aufzeichnungsmoduls, des Informationserkennungsmoduls, des intelligenten Moduls bzw. des Hämatomentfernungs- und -drainagemoduls mit Gleichspannung verwendet wird.

## Revendications

1. Système intelligent d'élimination et de drainage d'hématomes intracrâniens, comprenant un module d'entrée et d'enregistrement d'informations, un module de détection d'informations, un module intelligent et un module d'élimination et de drainage d'hématomes, dans lequel,
le module d'entrée d'informations est utilisé pour collecter des données préopératoires et transmettre les données préopératoires collectées au module intelligent,
le module de détection d'informations est utilisé pour acquérir des données intracrâniennes surveillées en temps réel et transmission les données intracrâniennes surveillées au module intelligent,
le module intelligent est utilisé pour recevoir les données depuis le module d'entrée d'informations et le module de détection d'informations, analyser les données reçues pour effectuer une détermination, et convertir la détermination en signal d'instruction et le transmettre au module d'élimination et de drainage d'hématomes, et
le module d'élimination et de drainage d'hématomes est utilisé pour recevoir une instruction depuis le module intelligent pour éliminer et drainer les hématomes intracrâniens, et pour renvoyer des données après l'élimination et le drainage au module intelligent, le système intelligent d'élimination et de drainage d'hématomes intracrâniens est **caractérisé en ce que**,
le module de détection d'informations comprend un module de détection d'images, un module d'acquisition de signaux et un module de détection de drainage, dans lequel, le module de détection d'images est utilisé pour détecter un état de l'hématome intracrânien et déterminer la forme de l'hématome intracrânien,
le module d'acquisition de signaux est utilisé pour acquérir des informations sur la pression intracrânienne et surveiller et transmettre les informations sur la pression en temps réel, et
le module de détection de drainage est utilisé pour détecter des composants de drainage par analyse en temps réel, détecter la composition des composants sanguins et surveiller les saignements en temps réel.

2. Système intelligent d'élimination et de drainage d'hématomes intracrâniens selon la revendication 1, dans lequel le module d'acquisition de signaux comprend un capteur de pression intracrânien et un module de conditionnement de signaux, le capteur de pression intracrânien est en communication avec le module de conditionnement de signaux, et le module de conditionnement de signaux est utilisé pour effectuer un traitement d'amplification non linéaire sur le capteur de pression intracrânien et transmettre le signal conditionné au module d'élimination et de drainage d'hématomes pour une opération.

3. Système intelligent d'élimination et de drainage d'hématomes intracrâniens selon la revendication 1, dans lequel le module de détection de drainage comprend un module de détection spectrale et un module de détection de couleur, qui transmettent respectivement des données à un système de contrôle principal, dans lequel le module de détection spectrale est utilisé pour détecter des informations spectrales, et le module de détection de couleur est utilisé pour détecter des informations de couleur.

4. Système intelligent d'élimination et de drainage d'hématomes intracrâniens selon la revendication 1, dans lequel le module intelligent comprend une base de données en nuage, un module d'analyse intelligent, un module de traitement de données et un module de transmission de signaux d'instruction, dans lequel la base de données en nuage est utilisée pour classer et stocker des données et mettre à jour les données stockées en temps réel,
le module d'analyse intelligent est utilisé pour comparer et analyser diverses données collectées avec les données d'origine dans la base de données en nuage,
le module de traitement de données est utilisé pour recevoir et transmettre les données intracrâniennes en temps réel, et
le module de transmission de signaux d'instruction est utilisé pour transmettre les signaux d'instruction.

5. Système intelligent d'élimination et de drainage d'hématomes intracrâniens selon la revendication 1, dans lequel le module d'entrée d'informations comprend un module d'informations de base et un module de description d'état pathogénique, dans lequel le module d'informations de base est utilisé pour saisir les informations de base du patient, et le module de description d'état pathogénique est utilisé pour saisir la description de base de l'état pathogénique du patient.

6. Système intelligent d'élimination et de drainage d'hématomes intracrâniens selon la revendication 5, dans lequel les données du module d'informations de base comprennent, sans s'y limiter, le nom, le sexe, l'âge, le lieu d'origine et le numéro de dossier médical du patient.

7. Système intelligent d'élimination et de drainage d'hématomes intracrâniens selon la revendication 1, dans lequel les modes de traitement du module d'élimination et de drainage d'hématomes comprend, sans s'y limiter, un mode d'aspiration simple à pression négative, un mode de drainage simple sans pression, un mode de remplacement et de drainage isobare, un mode de remplacement et de drainage par irrigation goutte à goutte et un mode d'injection d'une solution médicamenteuse à travers un canal de serrage de drainage.

8. Système intelligent d'élimination et de drainage d'hématomes intracrâniens selon l'une quelconque des revendications 1 à 7, comprenant en outre un module d'alarme et un module d'alimentation électrique, dans lequel,
le module d'alarme est utilisé pour émettre une alarme lorsqu'une intervention manuelle est nécessaire ou que le module d'entrée et d'enregistrement d'informations, le module de détection d'informations, le module intelligent et le module d'élimination et de drainage d'hématomes tombent en panne, et
le module d'alimentation électrique est utilisé pour fournir des tensions à courant continu au module d'entrée d'informations et d'enregistrement, au module de détection d'informations, au module intelligent et au module d'élimination et de drainage d'hématomes, respectivement.
